# EUROPEAN PATENT APPLICATION

(11) **EP 4 549 404 A1**
(43) Date of publication of application: **07.05.2025**
(21) Application number: 24210875.1
(22) Date of filing: 05.11.2024
(51) Int. Cl.: C02F 1/00, G01N 1/10, G01N 21/01, G01N 21/75, E04H 4/12, G01N 33/18, C02F 1/68, C02F 103/42

(54) **SYSTEMS, UNITS AND METHODS FOR MONITORING SWIMMING POOL CHARACTERISTICS BY MEASURING SWIMMING-POOL WATER-MANIPULATION RESPONSIVITY**

(30) Priority: 05.11.2023 US 202318502017
(71) Applicant: Maytronics Ltd., 1935000 Kibbutz Yizrael (IL)
(72) Inventor: Haviv, Shimry, Kiryat Tivon (IL); Tzur, Doron, Herzliya (IL)
(74) Representative: Kasche & Partner

(57) **Abstract**

Systems, units and methods for monitoring water of at least one swimming pool (SP) by: obtaining non-manipulation data of the SP water or one or more samples thereof before/without applying any manipulation to water of the SP sample(s) thereof; applying at least one manipulation over the SP water or one or more samples thereof; obtaining manipulation data of the SP water indicative of responsivity of the SP water or one or more samples thereof to each manipulation applied thereto, using one or more sensors; and analyzing the non-manipulation and manipulation data to determine one or more characteristics of the SP water including for example concentration of one or more substances in the SP water. In some embodiments, at least one manipulation may include dechlorination of the SP water.

## Description

### FIELD OF THE INVENTION

The present disclosure relates in general to systems and methods for measuring one or more swimming pool characteristics using one or more sensing and processing means, and more particularly to systems and methods for measuring swimming pool characteristics incorporating measuring the response of swimming pool (SP) water to one or more manipulations applied to the SP water or to one or more samples thereof.

### BACKGROUND

Swimming pool water require constant measurement and monitoring of various parameters, characteristics and properties such as chemical and/or biological/organic pollutants concentration, concentration of disinfecting materials and compounds, etc. in order to detect various swimming pool characteristics such as related characteristics/properties and maintain water quality and/or swimming pool device's functionality

Swimming pool (SP) water quality related properties such as concentration of one or more chlorine species, cyanuric acid concentration, pH level, turbidity level, biomass (algae/microalgae) concentration etc., which may impact bathers' health, should be measured on a regular basis. Swimming pool maintenance management and planning may also depend on values of the specific measured swimming pool water properties.

Cyanuric acid measuring is often used as a precursor for one or more chlorinated cyanurates such as anhydrous sodium dichloroisocyanurate, sodium dichloroisocyanurate dihydrate, trichloroisocyanuric acid, etc. Chlorinated cyanurates are recently administered to SP water mainly for minimization of sunlight chlorine degradation.

Measuring spectral properties of SP water may be used to detect/determine various water quality related properties such as chlorine species and/or biomass substances concentrations. However, some substances that may exist in the water may be difficult to spectrally distinguish from substances of interest such as different types of chlorine species thus rendering such optical measuring potentially unreliable.

Other measuring techniques (not necessarily optical) used to identify concentrations of several substances of one or more types in SP water have also been found as lacking desired reliability or require highly time-consuming, cumbersome, and/or expensive measures to reach desired accuracy and simplicity levels.

### SUMMARY

Aspects of disclosed embodiments pertain to a method for monitoring at least one swimming pool (SP), the method may include at least:
providing a detection unit that includes at least one sensor for measuring one or more properties of water of the specific SP;
providing at least one processing unit, configured at least to receive and analyze sensor data from the detection unit for determining one or more characteristics of the SP;
obtaining non-manipulation data of the SP water by measuring one or more properties of the SP water or at least one sample of the SP water, without it being manipulated, using the detection unit;
applying at least one manipulation over the SP water or at least one sample from the SP water, using at least one corresponding manipulation unit, the at least one manipulation including at least dechlorination of the SP water or at least one sample from the SP water;
obtaining manipulation data of the SP water, using the detection unit, indicative of a response of the SP water to the manipulation; and
receiving and analyzing the non-manipulation data and the manipulation data of the specific manipulation, at least for determining one or more chlorine species concentration in the SP water.

According to some embodiments, the at least one manipulation may be used for one or more of:
determining concentration of one or more substances in the SP water;
determining relations between one or more of SP water properties and one or more other SP water properties for the specific SP;
calibration and/or adjustment of one or more values of one or more SP parameters, associated with one or more SP properties, that are used for analyzing sensor data arriving from the detection unit, relating to the specific SP;
determining level and/or value of one or more parameters associated with water quality;
calibration and/or adjustment of one or more parameters values, thresholds and/or relations indicative of expected normal behavior of the specific SP.

According to some embodiments, the determination of concentration of one or more substances further includes determining concentration of one or more of: cyanuric acid; calcium; biomass of one or more types; one or more chloramines; one or more chloramines including one or more of: NH₂Cl, NHCl₂, NCl₃; one or more cyanurate species; one or more cyanurate species including one or more of: H₃Cy, H₂Cy⁻, HCy²⁻, Cy³⁻; one or more chlorinated cyanurates; one or more chlorinated cyanurates including one of more of:Cl3Cy, Cl2Cy⁻, HClCy⁻.

According to some embodiments, the determination of level and/or value of one or more parameters includes at least one of: pH level; alkalinity level, Free Chlorine Level, Combined Chlorine Level, Oxidation Reduction Potential (ORP) level, turbidity level, water hardness level.

According to some embodiments, the at least one manipulation includes introduction of one or more chemical reagent to the SP water or to one or more samples of the SP water.

The one or more chemical reagents may include one or more of:
one or more dechlorination reagents;
an acid of one or more acid types and/or of one or more concentrations;
one or more chlorination reagents of one or more types;
one or more chlorine species;
one or more dye substances;
one or more titrants;
one or more buffering substances;
one or more dye substances;
one or more halochromic materials;
one or more gassing and/or degassing materials.

According to some embodiments, the dechlorination manipulation includes one or more of:
introducing of one or more dechlorination reagents of one or more types into the SP water or one or more samples of the SP water;
heating of the SP water or one or more samples of the SP water;
applying pressure to the SP water or one or more samples of the SP water; and/or
bubbling of the SP water or one or more samples of the SP water.

According to some embodiments, the one or more dechlorination reagents include: Barium Sulfite and/or Calcium Sulfite, Sodium Sulfite.

The one or more dechlorination reagents may be in a form of dissoluble tablets.

According to some embodiments, the at least one manipulation includes one or more of:
actively heating of at least one sample of the swimming pool water for evaporating one or more substances from the at least one sample or for water evaporation and residue measurements;
actively or passively cooling of at least one sample of the SP water;
gassing and/or degassing at least one portion or at least one sample of the swimming pool water;
applying a physical force over the SP water or at least one sample thereof;
dying of the SP water or at least one sample thereof after the at least one reagent has been introduced to the SP water or at least one sample thereof.

According to some embodiments, the obtainment of the manipulation data is sone after a reaction time associated with the specific manipulation applied to the SP water or at least one sample thereof.

Aspects of disclosed embodiments pertain to a system for monitoring water quality related characteristics of water of a swimming pool (SP), the system including at least:
a detection unit including one or more sensors, the detection unit being configured for measuring at least one or more properties of the SP water;
a manipulation unit configured to apply one or more manipulations over the SP water or one or more portions thereof; and
at least one control and processing unit configured to receive and analyze sensor data arriving at least from the detection unit, where the detection unit and the at least one control and processing unit are configured to:
obtain non-manipulation data of the SP water by measuring one or more properties of the SP water or at least one sample of the SP water, without it being manipulated, using the detection unit;
obtain manipulation data of the SP indicative to response of the SP water to the applied manipulation, using the detection unit; and
analyze the obtained non-manipulation data and the manipulation data of the specific manipulation, at least for determining concentration of one or more chlorine species in the SP water.

According to some embodiments, the dechlorination of the SP water is done by at least one of:
introducing of a dechlorinating reagent of at least one type to the SP water or at least one sample from the SP water;
heating of the SP water or at least one sample from the SP water;
bubbling of the SP water or at least one sample from the SP water.

According to some embodiments, the analysis of the non-manipulation and manipulation data is further done for at least one of the following purposes:
determining concentration of one or more other substances in the SP water;
determine level and/or value of one or more parameters associated with water quality;
calibrating and/or adjusting one or more values of one or more pool characteristics, used for analyzing sensor data arriving from the detection unit, relating to the specific SP; and/or
calibrating and/or adjusting one or more curves and/or tables of values of one or more pool-characteristics, used for analyzing sensor data arriving from the detection unit.

The one or more sensors of the detection unit may include one or more of: at least one optical detector; at least one temperature sensor, at least one Oxidation Reduction potential (ORP) sensor, at least one pH sensor, at least one spectrometer, art least one camera, at least one photodetector.

According to some embodiments, the system may further include a sampling unit that comprises at least one sample cell, each sample cell being configured to obtain and hold therein a water sample sampled from the water of the SP, for conducting all measurements and manipulations over one or more water samples when held within the at least one sample cell.

According to some embodiments, the at least one sampling unit is also configured for filling and drainage of SP water into and out from each sample cell.

According to some embodiments, the system may further include one or more mixing mechanisms configured for mixing a reagent with a water sample obtained from the SP water.

According to some embodiments, the sample cell is at least partially transparent to enable illumination of the water sample and to enable optical detection of the obtained water sample, when held inside the sample cell, and where the system further comprises an illumination unit comprising at least: at least one light source, positioned and configured to illuminate the SP water, where the obtaining of the non-manipulation and manipulation data is done by measuring optical absorption properties of the SP water before and after and/or during it has been manipulated.

According to some embodiments, the dechlorination manipulation may include one or more of:
introducing of one or more dechlorination reagents of one or more types into the SP water or one or more samples of the SP water;
heating of the SP water or one or more samples of the SP water;
applying pressure to the SP water or one or more samples of the SP water; and
bubbling of the SP water or one or more samples of the SP water.

According to some embodiments, the system may also include a sampling unit which may include one or more sampling cells, where one or more a dissolvable tablets of at least one type of a dechlorination reagent are positioned: inside at least one of the one or more sample cells of the sampling unit; inside a reagent chamber having at least one controllable sealing door that can be controllably open and closed for controlling manipulation of a SP water sample held within the sample cell; and/or in at least one controllable reagent dispenser configured to controllably dispense doses of one or more reagents into one or more SP water samples or to the SP water.

According to some embodiments, the at least one manipulation may include introduction of at least one reagent to the SP water or to one or more samples thereof, where the at least one reagent comprises one of: Barium Sulfite, Calcium Sulfite, Sodium Sulfite, one or more chlorine species, one or more acids, one or more dye substances, one or more buffering substances.

According to some embodiments, the system may further include a main controller, operatively associated with all units and modules of the system and with the at least one control and processing unit, for one or more of:
receiving and analyzing manipulation and non-manipulation data from multiple SPs;
determining a maintenance plan for each SP based on per SP manipulation data, based on analysis results;
controlling monitoring and/maintenance of each of the SPs; and/or
accumulate multiple SP monitoring data and adjust monitoring and/or maintenance of each SP, based on analysis of accumulated monitoring data.

### BRIEF DESCRIPTION OF THE FIGURES

The figures illustrate generally, by way of example, but not by way of limitation, various embodiments discussed in the present document.

For simplicity and clarity of illustration, elements shown in the figures have not necessarily been drawn to scale. For example, the dimensions of some of the elements may be exaggerated relative to other elements for clarity of presentation of details thereof. Furthermore, reference numerals may be repeated among the figures to indicate corresponding or analogous elements. References to previously presented elements are implied without necessarily further citing the drawing or description in which they appear. The figures are listed below:
**Fig. 1** shows a schematic illustration of a local monitoring system for monitoring one or more properties of water samples obtained from a swimming pool (SP) water source, by monitoring response of the SP water to one or more manipulations applied to the SP water, according to some embodiments;
**Fig. 2** shows a schematic illustration of a local monitoring system for monitoring one or more properties of water samples using multiple measuring units, according to some embodiments;
**Fig. 3** schematic illustration of a system for monitoring a group of swimming SPs via a local monitoring system of each SP of the SP group, according to some embodiments;
**Fig. 4** is a flowchart, schematically illustrating a method/process for determining one or more characteristics of a SP, by measuring measurable one or more water parameters/properties of the SP water before the SP water has been manipulated and during or after it has been manipulated, according to some embodiments; and
**Fig. 5** is a flowchart, schematically illustrating a method/process for determining one or more characteristics of a SP, by measuring of one or more optical absorption properties of at least one water sample obtained from the SP, according to some embodiments.
**Fig. 6** schematically illustrates a measuring unit of a system for determining one or more SP characteristics, where the measuring unit includes a sampling unit and a manipulation unit that controllably introduces at least one reagent of one reagent type into a water sample from a SP by using a reagent chamber with a controllably sealable door, according to some embodiments; and
**Fig. 7** schematically illustrates a sampling unit and several manipulation units of a system for determining one or more SP characteristics, where each manipulation unit includes a different dispenser for controllably introducing different reagents into a sample cell of the sampling unit for applying one or more reagent-based manipulations to the SP water sample contained by the sampling unit, according to some embodiments.

### DETAILED DESCRIPTION

Embodiments of the present invention are aimed, *inter alia,* at enabling improved and efficient monitoring of one or more swimming pools by monitoring properties of swimming pool (SP) water of each SP such as concentration of one or more chemical and/or organic/biological substances such as concentration of: one or more chlorine species, calcium, cyanuric acid, etc., and/or concentration of biomass such as microalgae, etc. and optionally also for measurement/determination of additional properties of the SP and/or water thereof such as pH level, turbidity level, alkalinity level, water-hardness level, SP filter(s) functioning level, etc., by applying of one or more manipulations over water from the specific SP and measuring SP water response to this/those applied manipulation(s).

Some of these properties may be measurable/determined by applying one or more manipulations over: one or more samples of the SP water, an area of the SP water, and/or the entire SP water. One or more of these manipulations may include, for example, introducing one or more substances such as one or more chemical/organic/biological reagents to the SP water or one or more water samples thereof, for chemically reacting with the SP water, for detection or measurement or adding in detection or measurement or any other manipulation of one or more SP characteristic by detection or measurement of the response of the SP water to the reagent. The one or more SP related characteristics may include, for example, concentration of one or more substances in the SP water such as concentration of chemical and/or biological/organic polluting and/or disinfection substances such as concentration of one or more chlorine species, biomass (such as algae), calcium, cyanuric acid, etc., and/or for determining other water quality related properties such as alkalinity level, pH level, turbidity level, water-hardness level, oxidation reduction potential (ORP), etc.

The term "introducing" a reagent or a substance used herein may refer to contacting SP water or sample(s) thereof in any way or manner with the reagent(s) such as, for example, by way of dispensing, contacting, administering, directing SP water through solid tablet(s) of the reagent, etc.

The term "manipulation" of SP water used herein may relate to applying of any one or more treatments to the SP water or one or more samples thereof and/or to conditioning of the SP water or one or more samples thereof, such as introducing of one or more reagents, heating/cooling and/or evaporating of the SP water or a sample(s) thereof, applying physical force such as acoustical vibrations to the SP water, incubating one or more samples of the SP water for determining bacteria/algae types and/or biomass growth rates, titration of the SP water, electrolysis of the SP water, etc.

The term "SP water" may also refer to one or more samples and/or one or more portions of the water from the SP and/or to the entire water volume of the SP.

According to some embodiments, the one or more manipulation(s) applied to the SP water, may be used for any purpose such as for dying one or more compounds in the SP water such as by using halochromic material(s), for titration of the SP water, for forming larger molecules or suspendable materials, for forming compounds of other chemical and/or other optical properties that are more easily detectable by sensor(s) of the system, etc., for chlorination and/or dechlorination of the SP water, for decomposing one or more substances/molecules in the SP water, and the like.

The systems, units and/or methods of embodiments pertaining to the present invention, may be designed to enable obtaining measurement(s)/information/data indicative of the SP water before, after and optionally during the SP water manipulation, using non-manipulation and manipulation measured data to determine one or more properties of the SP water such as: (i) concentration of one or more chemical and/or biological substance; (ii) values of one or more physical properties of the SP water such a pH level, alkalinity level etc.; (iii) one or more SP facility devices' functionality measures; and/or (iv) one or more per-swimming pool personal baseline measures of the specific SP, such as the specific mathematical relation between one or more chlorine species concentration of the SP water to the Oxidation Reduction Potential (ORP) of the specific SP, specific, per SP, relation between pH level and alkalinity level, etc., and/or normal ranges and/or thresholds for the specific SP such as normal ranges for concentration of one or more substances such as one or more chlorine species, calcium, cyanuric acid, normal ORP range/threshold value(s), normal pH level, normal alkalinity level, normal temperature range, etc.

According to some embodiments, the term "non-manipulation" may refer to the SP water before or without any manipulation was applied thereto. This means that the SP water manipulation and obtainment of post-manipulation data indicative of SP water property(ies) responsivity to the applied manipulation, can be obtained after or during the applying of the respective manipulation. The pre and post manipulation data/measurements may be obtainable by using the same SP water sample, for example therefore requiring the non-manipulation data is measured before the application of the respective manipulation. Alternatively several (two or more) SP water samples may be used, where one of the samples is maintained unmanipulated such that pre and post manipulation measurements/data can be simultaneously obtained.

According to some embodiments, using several separate sampling units/cells, where one sample is remained unmanipulated, may also enable quick and/or simultaneous measurement of SP water responsivity to multiple different manipulations e.g., by applying a different manipulation to each of the other samples (other than the unmanipulated one).

The manner of manipulation and subsequent manipulations may itself be partially or entirely determined by results obtained from previous manipulations.

According to some embodiments, the determined SP characteristics and/or SP water properties of each specific SP, may be used to further determine maintenance related actions and/or details/parameter values thereof as well as scheduling of such maintenance actions, such as specific quantities and schedules for dispensing/introducing of one or more additive materials, such as disinfection materials, and timing and schedules of operation of various maintenance related devices such as schedules and mode-selection (such as standard or backwash) of operation of one or more filters of the SP, quantities and schedules of SP water filling and/or drainage, and time and manners of subsequent manipulations etc.

According to some embodiments, at least one such manipulation may include introducing a chlorinating or a dechlorinating reagent to a water sample of the SP, for chlorinating or dechlorinating the SP water sample(s) and measuring the water sample before and after and/or during the chlorination/dechlorination of the sample. According to some embodiments, a few SP water samples may be used where one is not applied to with any manipulation and one or more other samples are applied to with one or more different manipulations, such that the "pre-manipulation" data is obtained by measuring one or more properties of the non-manipulated sample, using one or more sensors/detectors, and the "post-manipulation" data is obtained by measuring corresponding properties of the one or more additional samples during or after the manipulation thereof. The impact/influence of each manipulation made, may be determined by comparing the properties of the non-manipulated sample with corresponding properties of each of the one or more manipulated samples.

The measuring of the SP water sample may include measuring optical properties of the water sample such as absorption properties (absorption lines or absorption spectra) of the water sample before, after and optionally also during its manipulation (e.g., dechlorination). The comparison between the non-manipulation absorption properties of the sample and its absorption behavior during and/or after the manipulation, as well as the dynamics of the changes, may enable improved detection/determination and distinction of substances concentration, such as (in the case of a dechlorination manipulation) improved spectral distinction of several different types of chlorine species in the SP water, which would otherwise would be difficult if not impossible to spectrally distinguish from one another.

According to some embodiments, such chlorine species may include free chlorine species such as Hypochlorous acid HOCl, hypochlorite ions OCL⁻and their ratios. Since higher relative concentration of Hypochlorous acid HOCl is associated with a lower pH level, the concentration of Hypochlorous acid may be also used to estimate the pH level of the SP water.

Other additional or alternative measurable/detectable (e.g., by way of optical/spectral/absorption properties' detection and/o analysis) concentration of chlorine species may include, for example, chlorine Cl₂, and/or chloramines such as NH₂Cl, NHCl₂, NCl₃, and/or concentration of other substances such as concentration of cyanurate species such as H₃Cy, H₂Cy⁻, HCy²⁻, Cy³⁻ and/or chlorinated cyanurates such as Cl3Cy, Cl2Cy⁻, HClCy, etc.

According to some embodiments, after the dechlorinating reagent is introduced to the SP water or to one or more samples thereof, the influence of the SO₃ ions on the spectra of the SP water is determined/calculated (e.g., based on comparison between the pre and post dechlorination spectral measurements and optionally also potentially based on compensating for the SO₃ ions concentration in respect to SP water (detectable) temperature). By comparing the pre and post dechlorination spectral (e.g., absorption) properties of the SP water, the impact of the SO₃ over the SP water can be spectrally measured. In some embodiments, the measured SO3 concentration may be further compered to a desired or expected SO3 concentration enabling manual or automatic compensation for detected low SO3 concentration. According to some embodiments, the expected or desired SO3 concentration may be dependent on measured or estimated temperature of the SP water.

Additionally or alternatively, once a dechlorinating reagent is introduced to the SP water sample and chemically reacts with the SP water, residual byproduct chlorine species may still be present in the SP water sample requiring filtering, distillation or separation thereof e.g., by heating and evaporating at least volatile such reaction byproducts, for achieving a manipulated sample that is of a significantly reduced or free from these byproducts, such that the chlorine species concentration of the SP water can be measured by comparing the cleaner dechlorinated water sample absorption properties with absorption properties of the unmanipulated water sample (obtained or measured before the dechlorination of the water sample).

Additionally or alternatively, no filtration or distillation of the SP water or sample(s) thereof is performed, where the chlorine species are detectable due to their spectral differentiation from the dechlorination byproducts and/or from other substances in the SP water.

In some cases, the byproducts of the reaction of the SP water with the reagent(s) are more distinguishable by the one or more sensors, such as more optically (e.g., spectrally) distinguishable, and therefore do not require filtration thereof but can be identified and accounted for algorithmically.

In some (potentially other or additional) cases, the chemical/biological byproducts of the applied manipulation may be heavier or lighter than SP water and therefore sink to a bottom of the sample cell or the SP or float to the top of the sample cell, enabling acquiring optical (e.g., spectral/absorption) measurements of the SP water before and after (and optionally also during) the applying of the respective manipulating thereover, requiring a reaction and sinking (or floating) time for measuring the SP water sample after the byproducts are estimated to sink and reach the bottom of the sample cell or the SP or float and reach the top of the sample cell of the SP.

In some (potentially other or additional) cases, the byproducts of the reaction of the SP water with the reagent(s) are the substance(s) required to be measured, requiring measuring of the SP water shortly after the reaction time is ended to capture them before inking to the bottom of the sample cell or the SP or alternatively applying gentle stirring to keep them suspended in the SP sample.

According to some embodiments, the one or more reagents being used may be in any form such as in the form of part of a structural member made of the material, dissolvable tablets, powder, liquid and/or any state such as solid, liquid etc. In case of a reagent used for applying a dechlorination manipulation, one or more dechlorinating salts (e.g., in a form of dissolvable tablets) may be used, including for example, one or more of: Barium Sulfite, Calcium Sulfite and/or Sodium Sulfite.

Aspects of disclosed embodiments pertain to a method for monitoring a swimming pool (SP) for determining one or more characteristics of the SP such as concentration of one or more substances in the SP water, SP water quality related parameters' values, and/or SP water quality related threshold ranges/values and/or behavioral basis and/or relations specific to a certain SP that may be advantageously used to monitor and understand its behavior. The monitored water quality related properties of the SP may enable determining associated SP characteristics such as SP devices functionality (such as filter(S) functionality), pollution status of the SP etc. and/or enable to determine required actions such as alerts issuance and/or initiation of maintenance related actions for the specific SP, based on determined SP characteristics.

According to some embodiments, there is provided a dechlorinator, which includes a dechlorinating unit configured to introduce dechlorination reagent such as barium sulphite or calcium sulphite, to the SP water or to one or more samples thereof and optionally also detect one or more SP water properties such as spectral properties of the SP water before and after the dechlorination thereof, SP water temperature etc.

The dechlorinator may be embedded in any monitoring system or any other apparatus/device that is designed to take SP water samples and/or to be submersible in the SP water of the SP such as embedded in a skimmer, a SP cleaning robot, a floatable apparatus, a SP monitoring system etc.

According to some embodiments, the dechlorinator may detect one or more of the following SP water properties before and after the SP water dechlorination using a designated one or more sensor(s)/detector(s):
spectral properties of the SP water (e.g., using a spectral/optical detector);
ORP of the SP water (e.g., using an ORP sensor);
Chlorine level using a chlorine sensor;
pH properties (e.g., using a pH detector).

According to some embodiments, the method may include:
providing a local monitoring system for a specific SP;
obtaining at least one water sample from the water of the specific SP e.g., by channeling water from the SP into one or more sample cells of at least one sampling unit of the local monitoring system;
illuminating the water sample e.g., by using at least one illuminator of an illumination unit of the local monitoring system;
measuring non-manipulation properties of the water sample, using one or more sensors of a detection unit of the local monitoring system such as by using one or more detectors/sensors of the detection unit such as a spectral detector, a ORP sensor etc. and/or a pixelated optical sensor/camera, one or more temperature sensors, one or more pH sensors, one or more bolometric sensors, one or more pressure sensors, one or more piezoelectric transducers, etc., thereby obtaining sensor data associated with, indicative of, or that is non-manipulation.
data from at least one of the one or more sensors of the detection unit;
applying one or more manipulations to one of the obtained one or more water samples (which could be the same water sample used for the non-manipulation measurements or a different sample of un-manipulated water taken from the same SP at a proximate time compared to the measuring of the non-manipulation data), optionally by introducing at least one reagent of at least one reagent-type to the water sample of the SP water, thereby obtaining manipulation data; and
receiving and analyzing non-manipulation data and manipulation data from the detection unit (e.g., associated with a same specific "manipulation session") to determine one or more SP water properties and/or SP characteristics of the specific SP such as: concentration of one or more substances in the SP water, such as concentration of one or more polluting and/or disinfecting substances such as concentration of one or more chlorine species, concentration of cyanuric acid, concentration of biomass, concentration of calcium etc., and/or value/level of one or more parameters associated with water quality of the SP water such as pH level, ORP level, alkalinity level, turbidity level, water-hardness level, water purity level, etc.

According to some embodiments, the terms "non-manipulation data" and "manipulation data" used herein may refer to updated sensor data outputted from one or more sensors of the detection unit of the local monitoring system used for sensing of the SP water or sample(S) thereof respectively before the application of the respective manipulation (for the "non-manipulation data") and after and/or during the applying of the specific manipulation (for the "manipulation data").

According to some embodiments other received and/or measurable properties of the SP water and/or of the SP may be taken into account for determining associated SP characteristics, by combining the additional measurable/received SP properties with properties measured before and during and/or after the applying of each manipulation to the SP water such as measured updated water temperature (e.g., using one or more temperature sensors of the detection unit), known properties of the specific SP such as general or known current volume of its water, estimated number of bathers and/or activities occurring in the SP, known amounts of recently added one or more disinfection additives and/or water and timing of their administration, updated/current ambient temperature of the specific SP surrounding, known or estimated sunlight illumination of the specific SP, etc.

The term "dechlorinating", or any other grammatical conjugation thereof, may refer to any process in which chlorine atoms and/or chlorine-based compound(s), molecules and/or ions in the SP water interact with the reagent (e.g., by chemically bonding the dechlorinating reagent or part(s) thereof to chlorine atoms, chlorine-based molecules/ions and/or other substances in the water sample) or any other method of reducing the chlorine concentration in the SP water e.g., by heating, bubbling the SP water etc..

According to some embodiments, there is provided a system such as a local monitoring system, for monitoring water quality related characteristics of water of a specific swimming pool (SP), the system may include at least:
a detection unit comprising one or more sensors, the detection unit being configured for measuring one or more properties of SP water;
a manipulation unit configured to apply one or more manipulations over the SP water or one or more portions thereof, optionally by introducing at least one reagent to water from the SP, at each manipulation-session; and
at least one control and processing unit configured to receive and analyze sensor data arriving at least from the detection unit to determine one or more SP water properties.

According to some embodiments, the detection unit and the at least one control and processing unit may be configured at least to:
measure the SP water before applying any manipulation to the SP water, for obtaining non-manipulation data of the SP water, indicative of non-manipulation properties of the SP water, using the detection unit;
measure the SP water after and/or during its manipulation at least for obtaining manipulation data of the SP water; and
analyze the non-manipulation data and the manipulation data of the specific manipulation, at least for determining one or more one or more SP water properties.

According to some embodiments, the determined one or more SP water properties may be also used to determine one or more SP characteristics.

Aspects of disclosed embodiments may pertain to a system for measuring one or more properties of SP water of a specific SP. This system may include, for example one or more measuring units, at least one of the measuring units including, for example:

At least one sampling unit comprising at least one sample cell, each sample cell being configured for holding therein a water sample, obtainable from the specific SP;
(optionally - in case optical measurements are required) at least one illumination unit including at least one light source (illuminator) positioned and configured to illuminate the water sample in the sample cell;
at least one detection unit that includes one or more sensors such as (yet not limited to) at least one optical detector enabling detection of spectral characteristics such as absorption lines or spectra of SP water and one or more compounds in such water, a temperature sensor, a ORP sensor etc.;
at least one manipulation unit configured for applying one or more manipulations of one or more manipulation types, over the water sample(s) held in the sample cell(s) e.g., by introducing one or more reagents to the water sample(s);
at least one control and processing unit for receiving and analyzing sensor data from the detection unit for determining one or more SP water properties of the SP, based on non-manipulation and manipulation data processing/analysis of the respective manipulation and optionally also based on non-manipulation and manipulation data of other manipulation of the same and/or different manipulation type.

Each new water sample filled into the sample cell of the sampling unit may be measured before any manipulation is applied thereto for obtaining updated non-manipulation data of the SP water for the specific manipulation session to be conducted by the system. After the non-manipulation data is obtained, the same or any other water sample taken from the same SP may be manipulated by introducing one or more reagents of one or more reagent types to the water sample (contained within the sample cell) or by other manipulation means such as heating, filtering, electrolysis etc., and measuring the sample's reaction/responsivity to the administered reagent(s) or applied other manipulation - while obtaining sensor data indicative of the manipulation data of that specific applied reagent-based manipulation.

The non-manipulation and manipulation data can then be analyzed (e.g., by way of comparing these two data types indicative of experimental results of the SP water's responsivity to the applied reagent-based manipulation, to determine, based on this data analysis, one or more SP characteristics such as concentration of one or more polluting and/or disinfecting substances, parameters level/value, etc.

According to some embodiments, the system may be configured to carry out automatic/controllable periodic sampling and testing/measuring of SP water based on programmable monitoring routine(s)/plan(s), and/or in response to user requests and/or if certain conditions occur (for example after heavy rain or if previous results indicated rapid change in SP parameters). According to some embodiments, a managing application can be used operable via end devices, for displaying measured and calculated/ determined SP water properties , enabling to operate one or more of the SP devices, etc.

In some embodiments, a managing application operable via one or more user devices may enable one or more of: managing control of measurements processes, receive and display measurements results, enable controlling maintenance operations, set parameters values and/or thresholds etc.

According to some embodiments, the control and/or processing units of the systems of embodiments described herewith, may use any adapted software and/or hardware means at least to receive and analyze the sensor data for determining one or more water properties of each SP at each given moment/timeframe/period.

According to some embodiments the control and processing unit(s) of the local and/or remote system(s) may be configured to apply one or more artificial algorithms/modules for determining values of the one or more water properties and/or to determine which manipulations to apply and/or when and also optionally to schedule and control one or more SP devices' operations such as back wash, filtering operation, SP filling/drainage operations, etc.

According to some embodiments, the sampling unit may be configured to direct water from the SP into the sample cell and optionally also for drainage of the water sample from the sample cell once a measuring session thereof is completed such as to allow frequent and/or ongoing obtainments of new samples (one for each measurement session) and easy drainage thereof.

For example, the sampling unit may include or use one or more pumps, valves and/or pipes for pumping and directing water samples from the specific SP into each sample cell of the sampling unit as well as means for draining each acquired water sample from the sample cell.

According to some embodiments, the system may include one or more manipulation units, at least one of which may include one or more mechanisms for applying one or more other manipulations over the water sample for other separate quality/state measurements of the water sample that may be conducted separately from the dechlorinating measurements or combined therewith. For example, the system may additionally include one or more ultrasonic (US) transducers for applying of ultrasonic signals over the water (water) sample when in the sample cell for measuring turbidity of the sample and/or for cleaning of the sample cell's inner wall(s) and/or a de-bubbling device for de-bubbling the water (water) sample before any optical measurement thereof is conducted.

In some embodiments, the one or more US transducers may be used for improving or enhancing mixing of a reagent(S) with a water sample.

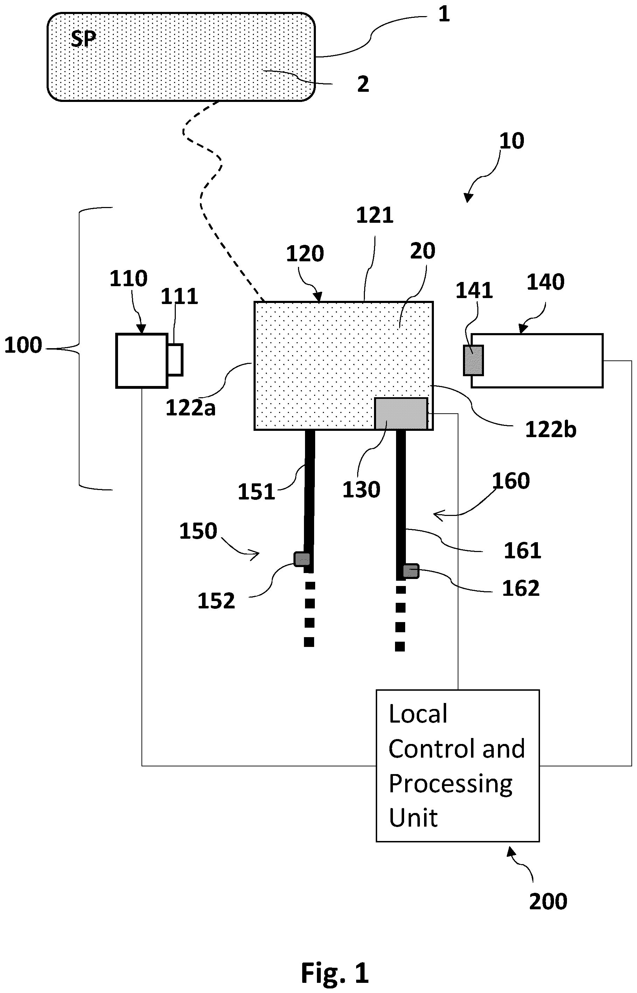

Reference is now made to **Fig. 1****,** showing a schematic illustration of a local monitoring system (LMS) **10** for monitoring one or more properties of one or more water samples obtained from a swimming pool (SP) **1** water source, by monitoring response of the SP water **2** to one or more manipulations applied to the SP water, according to some embodiments.

The LMS **10** may include:
one or more measuring units, such as measuring unit **100;** and
a local processing and control unit **200,** configured to receive and analyze/process data arriving from each of one or more measuring units **100** of the LMS **10** of the specific SP to determine one or more properties of the SP water **2** and SP characteristics of the specific SP **1** optionally to identify SP state and exceptions/hazards and optionally also to: control SP **1** facility/devices for operating, scheduling and/or planning ongoing maintenance of the SP **1**; and/or issue and/or send alerts upon identification of each hazardous or exceptional situation, based on analysis/processing results; and/or modify its own operation protocol, based on analysis/processing results.

According to some embodiments, each measuring unit **100** may include, for example:
an illumination unit **110** including one or more illuminators **111** such as one or more light emitting diode (LED) and/or one or more excimer light sources designed to emit light within a substantially narrow bandwidth within the ultraviolet (UV), visible or IR light ranges (such as only within one of the short-wave, middle-wave or long-wave UV ranges) and/or one or more wideband light sources such as a xenon gas discharge lamp or a tungsten incandescent lamp and optionally also one or more optical elements and/or optical devices for controlling the illuminator(s) and/or for optically manipulating light emanating from the one or more illuminators **111** of the illumination unit **110**;
a sampling unit **120** including at least one sample cell such as sample cell **121,** configured for receiving and holding therein water samples 20, sampled from the specific SP water **2** of the specific SP **1**;
one or more manipulation units such as manipulation unit **130** configured to manipulate each water sample **20** contained in the sample cell **121**; and
a detection unit **140** including one or more sensors such as an optical detector **141.**

According to some embodiments, the sample cell **121** may include one or more transparent surfaces and/or walls such as transparent walls **122a** and **122b,** for enabling light emanating from the illumination unit **110** to transfer through the sample cell **121** for illuminating the water sample **20** held therein, and for light passed through the sample cell **20** to be detected by the optical detector(s) **141** of the detection unit **140.**

According to some embodiments, the sample cell **121** includes a cuvette.

According to some embodiments the local processing and control unit **200** may include and/or use hardware and/or software means enabling digital data communication, storage, processing/analysis etc. and may be operatively associated with any one or more of the other LMS **10** units, for any one or more of:
receiving and/or transmitting of data therefrom/thereto;
controlling one or more devices of the LMS **10**;
processing data received from the detection unit **140** of each measuring unit 100;
receive and/or send data to one or more remotely located end devices or systems.

According to some embodiments, the detection unit **140** may include one or more of:
at least one optical detector comprising, for example, a camera, a pixelated photodetector, an array of photodetectors, a spectrometer, etc.,
at least one chemical sensor;
at least one pH sensor,
at least one ultrasonic (US) transducer;
at least one electrochemical sensor;
at least one halochromic sensor;
at least one Oxidation Reduction Potential (ORP) sensor;
at least one temperature sensor;
at least one bolometric sensor;
at least one conductivity sensor.

According to some embodiments, the detection unit **140** and/or the local processing and control unit **200** may be configured to detect spectral (absorption) optical properties of the water sample **20** held in the sample cell **121,** for example in order to detect/determine/estimate concentration of one or more chemical and/or organic/biological substances in the water sample **20.**

According to some embodiments, the LMS **10** may be configured such that the one or more sensors **141** of the detection unit **140** are automatically, electronically, and/or manually controllable to acquire sensor data of the water sample **20** in the sample cell **121** before any manipulation is applied thereto for obtainment of "non-manipulation data" of the water sample **20** as well as measuring response of the water sample **20** to the specific applied manipulation e.g., by measuring sample properties during and after the manipulation was applied optionally for a preset specific manipulation period Δt, for obtainment of the "manipulation data" and transmit this non-manipulation and manipulation data of the specific manipulation session to the local processing and control unit **200,** which may then process this received data e.g., by comparing non-manipulation and manipulation data, to determine one or more properties of the SP water and/or one or more SP characteristics such as concentration of one or more substances such as concentration of one or more chlorine species, concentration of cyanuric acid, etc.

According to some embodiments, some of the SP water properties and/or SP characteristics may be deduced from values of parameter-properties determined based on sensor data processing. For example, pH level measurements may be used to deduce alkalinity level.

According to some embodiments applying a dechlorination manipulation to the water sample **20** may enable deducing or estimating free chlorine levels from ORP level/value of the SP water **2.**

According to some embodiments, the spectral (absorption) optical measurements of the water sample **20** may be done by using one or more illuminators, each emitting light of a specific narrow wavelength (WL) bandwidth and a pixelated optical detector or a spectrometer or a photodetector, enabling separate detection of absorption properties of the pre-manipulated and post-manipulated water sample for each specific WL either due to the detector ability to differentiate WL or by selectively operating each illuminator while the others are turned off.

According to some embodiments, to determine/detect concentration of each chlorine specie from a predefined list of such specie, using the dechlorination manipulation(s), the SP water sample **20** is first measured by using an optical detector before any manipulation is applied thereover, to determine spectral properties of the pre-manipulated water sample **20** still containing therein (e.g., in a suspended state) all chlorine species in their current concentration; then a dechlorinating reagents) is introduced to the water sample **20** in the sample cell **121** and the sample **20** is measured again for spectral properties thereof after a predefined suspension time period for allowing the dechlorinating reagent(s) to chemically fully interact with the water sample **20** and optionally also for enabling the reaction byproducts to be filtered/evaporated/sink, before the water sample **20** is remeasured at its "post-manipulation"/"post-dechlorination" state.

According to some embodiments, dechlorination of the water sample may also enable measuring or estimation of free chlorine levels from an ORP value (by measuring the ORP of the sample when including approximately zero chlorine) and/or enable calibration of a chlorine sensor of the detection unit or of other devices of the SP from its value at approximately zero chlorine.

In some embodiments, ORP may be measured while gradually introducing chlorine reagent/disinfection additive to the SP water **2** or to the SP water sample **20** and measuring (for each dosage) ORP e.g., for measuring sensitivity of each of these sensor's and/or for generating personal calibration curves, per SP system, indicative of sensor output data and its correlated calibrated parameter value such as free chlorine concentration value, or cyanuric acid concentration value.

In some embodiments, manipulations of chlorine species concentrations and of acid concentration, together with pH measurement of a water sample measurements can also be used to determine/calculate alkalinity level of the SP water **2.**

According to some embodiments, one or more manipulations to one or more water samples for a SP may also be used to find a personalized dynamic range for values of one or more parameters, for the specific SP and/or for finding balanced working ranges/points for one or more parameters, which may improve maintaining the specific SP in a balanced state in which all/most of its water quality parameters values are within a personalized normal range that is appropriate for the specific SP but not necessarily for other SPs.

According to some embodiments, the manipulation unit(s) **130** may include one or more manipulation devices, elements etc. and may each be designed for application of one or more manipulations (e.g., of the same type such as reagent-introduction based manipulations and/or of different types e.g., physical manipulations such as heating, irradiating with US energy, illumination or filtration). For example, the manipulation unit may include one or more containers or dispensers for enabling administering one or more reagents into the sample cell **121.**

According to some embodiments, as shown in **Fig. 1****,** the water from the SP **1** may be directed/channeled to the sample cell **121** via a water filling subsystem **150** one or more pipelines such as pipeline **151** using one or more pumps, faucets and/or controllable valves such as controllable valve **152.**

According to some embodiments, as shown in **Fig. 1****,** each sample cell **121** of the sampling unit **120** may also include or connect to a drainage subsystem **160,** for draining water samples and emptying the sample cell **121.** The drainage subsystem **160** may include one or more drainage channels/pipes such as drainage pipe **151** and one or more controllable facets and/or valve such as controllable drainage valve **162.**

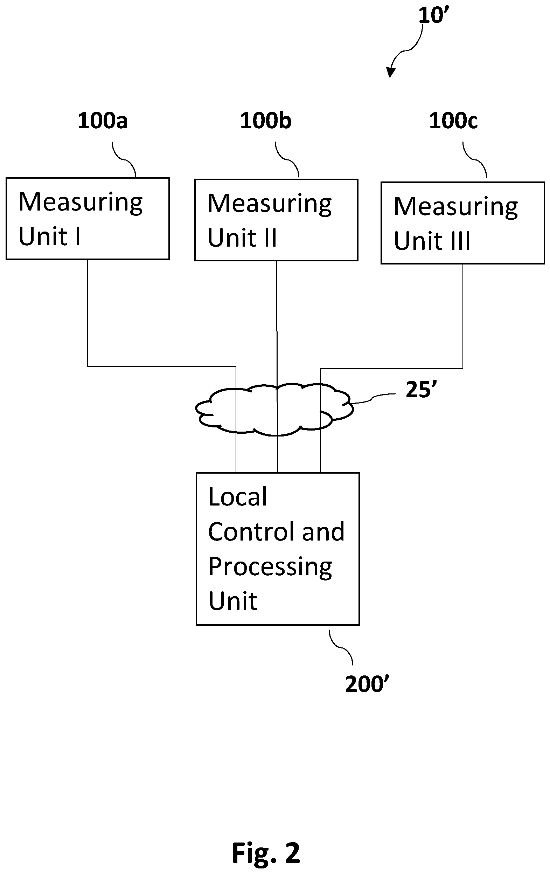

**Fig. 2** shows a schematic illustration of a local monitoring system (LMS) **10'** for monitoring one or more properties of water samples using multiple measuring units **100a-100c,** according to some embodiments, where each measuring unit **100a/110b/100c** may be configured for: a different set of manipulations; a different sampling technique and/or equipment; and/or for different measuring techniques. A local processing and control unit **200'** of this LMS **100'** may communicate with each measuring unit **100a/100b/100c** at least for receiving sensor data thereof, at least via one communication network, technique and/or hardware **25'.**

According to some embodiments, each LMS **10/10'** may use and/or include filling and emptying (drainage) devices and/or elements for filling and draining each sample cell with new samples from the respective SP.

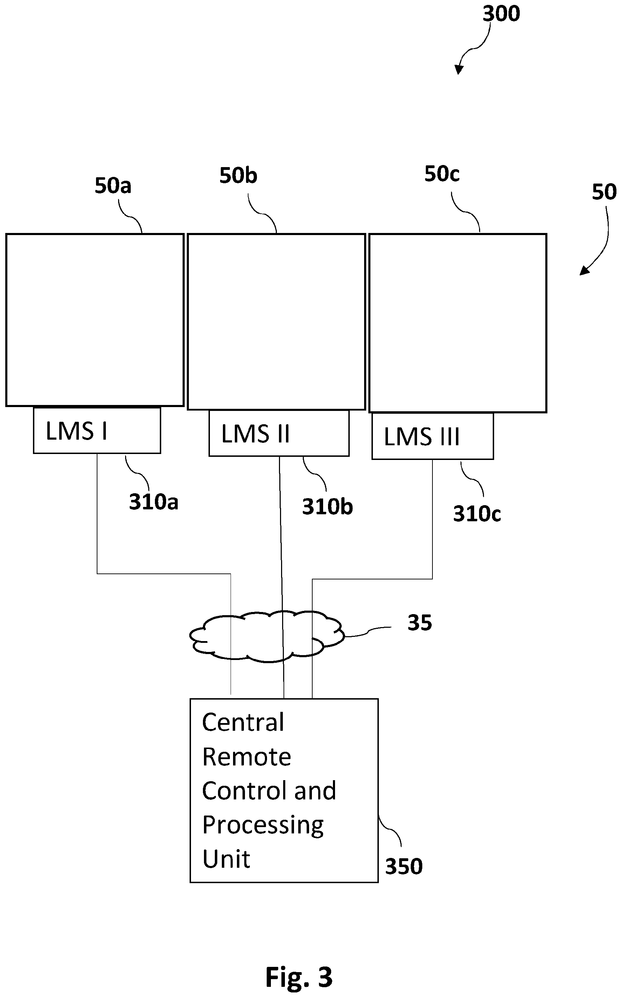

**Fig. 3** schematic illustration of a system **300** for monitoring a group **50** of swimming SPs via a LMS **310a/310b/310c** of each SP **50a/50b/50c** of the SP group **50,** according to some embodiments, using a remotely located central control and processing unit **350,** which can communicate with a local processing and control unit of each of the LMSs **310a/310b/310c** of the SPs **50a/50b/50c** of the SP group **50** via one or more communication channels such as communication channel **35.** The central control and processing unit **350** may be configured to carry out one or more of:
processing sensor data from each detection unit of each LMS;
control each LMS;
control other PS devices such as filtering, backwash, disinfection maintenance action etc.; and/or
determine schedules and quantities of maintenance actions for each SP **50a/50b/50c.**
learn from its history in order to improve and optimize the way it performs its tasks.

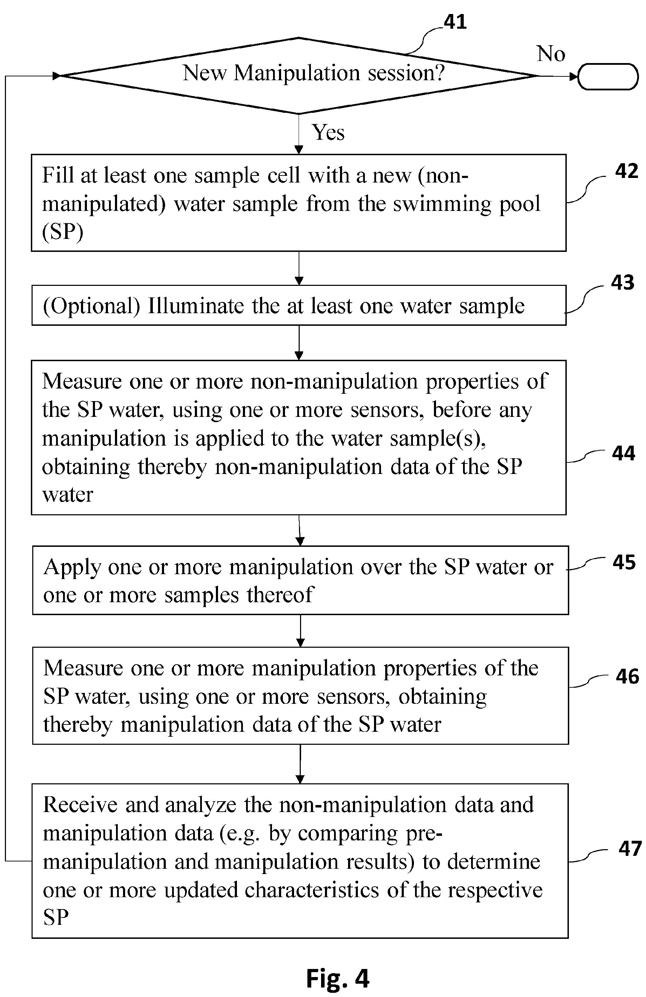

Reference is now made to **Fig. 4****,** which shows a flowchart, schematically illustrating a method/process for monitoring SP water using SP water manipulation(s) for determining water quality related properties of the SP, according to some embodiments.

The method may include at least some of the following steps:

For each new manipulation **41**:
filling at least one sample cell with a new water sample from the respective SP **42** (e.g., using one or more pumps, controllable valves and/or pipes for pumping and directing SP water from the SP to the sample cell(s);
(optional - for cases including optical detection) illuminating the water sample(s) **43**;
measuring one or more properties of each water sample before any manipulation is applied thereto for obtaining "non-manipulation data" of each sample **44**;
applying at least one manipulation over at least one water sample **45** e.g., at least by, for example, introducing at least one reagent to at least one water sample;
measuring one or more corresponding properties of each water sample during or after the application of each manipulation for obtaining "manipulation data" of each sample **46**; and
receiving and analyzing non-manipulation and manipulation data of each sample for each manipulation for determining/calculating/estimating one or more (updated) water quality properties of the SP water and/or other SP characteristics **47.**

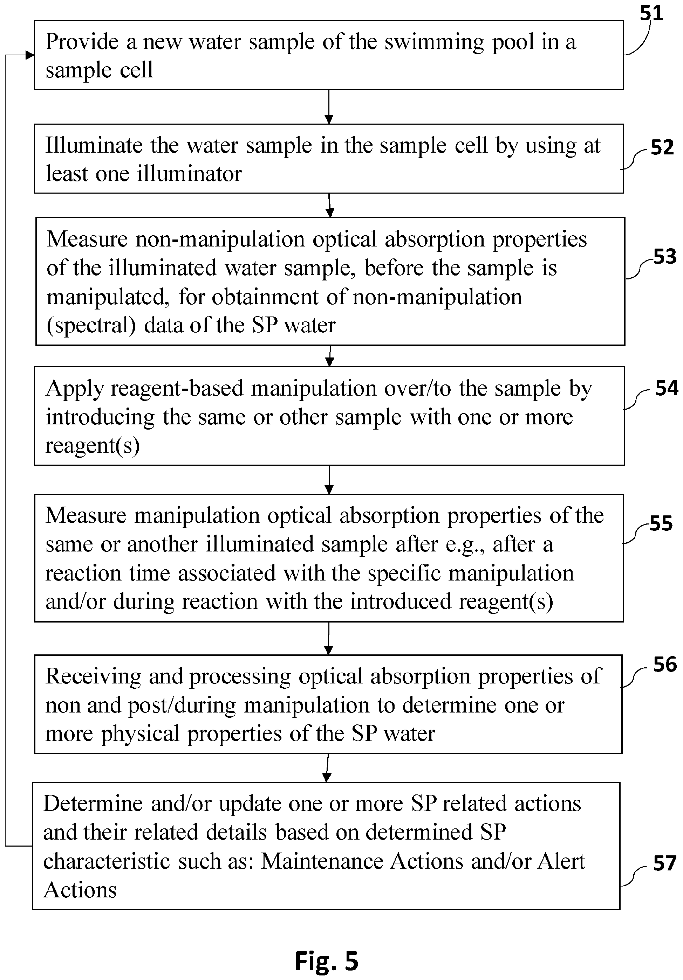

Reference is now made to **Fig. 5****,** which shows a flowchart, schematically illustrating a method/process for monitoring optical absorption properties of SP water using SP water manipulation(s) for determining water quality related properties of the SP, according to some embodiments.

The method may include at least some of the following steps:
providing a new water sample from a SP (step **51**):
illuminating the water sample by illuminating the sample cell e.g., using illuminator(s) of a narrow WL bandwidth, optionally operating one at a time (step **52**);
measuring one or more optical properties of the water sample, including at least spectral (absorption) properties of the water sample, before any manipulation is applied thereto for obtaining "non-manipulation data" of the sample (step **53**);
applying at least one reagent-based manipulation over the water sample (step **54**) by introducing one or more reagents of one or more reagent-types to the water sample;
measuring one or more corresponding (absorption) spectral properties of the water sample during or after the application of each manipulation for obtaining "manipulation data" of each sample (step **55**) for example by measuring the sample after a suspension period in which the water sample is estimated to fully react with the reagent;
receiving and processing optical absorption properties of the sample by comparing its spectral properties before and after the reaction thereof with the introduced reagent(s) (step **56**) e.g., to determine one or more SP characteristics; and
determine and/or initiate one or more actions (e.g., based on above processing and comparison results) such as maintenance action(s) and/or issuance/sending of alert(s) or information to one or more end devices or to a central control and processing unit (step **57**).

According to some embodiments, the manipulation unit of a LMS may include means for introducing the reagent(s) to the water sample in a controllable manner, by way of dispensing of a powder/liquid reagent into the sample cell of the sampling unit, or into the PS water or by way of directing water from the SP to be passed through the reagent(s) or by way of bringing the water in selective contact with a surface element made of or containing the reagent

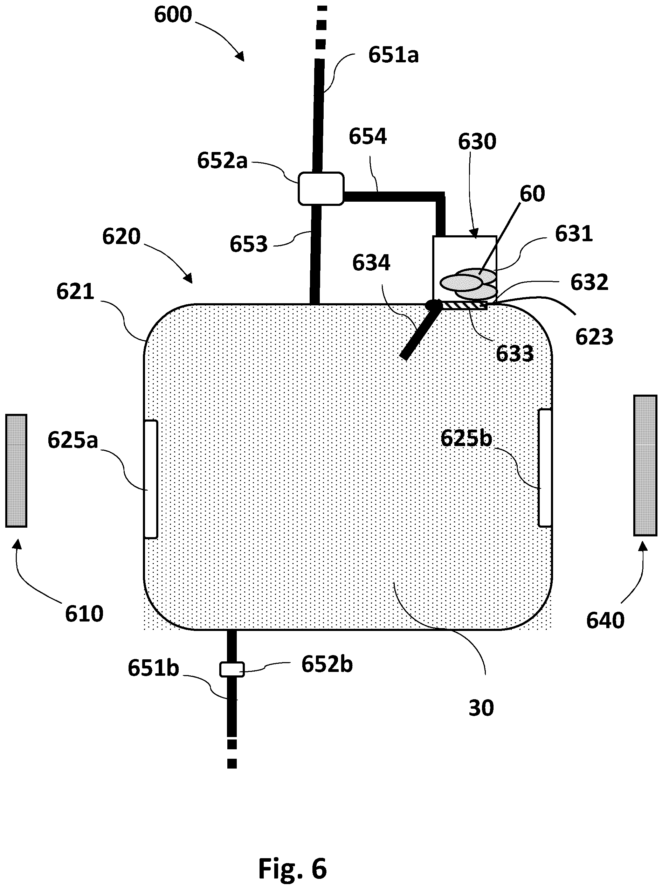

**Fig. 6** schematically illustrates a measuring unit **600** for measuring one or more SP water properties, according to some embodiments. The measuring unit **600** may include a sampling unit **620** and a manipulation unit **630** that controllably introduces at least one reagent of at least one reagent type to a water sample **30** from a SP, according to some embodiments.

The manipulation unit **630** may include a reagent chamber **631,** for containing therein one or more solid dissolvable reagent tablets such as tablets **60,** where the reagent chamber **631** may be located such that, it engages an opening **623** of the sample cell **621.** The reagent chamber **631** may also include a controllable sealing door **634** that controllably opens and seals an opening **632** of the chamber **631** that is located such as to overlap opening **623** of the sample cell **621.** A barrier **633** such as a bar/grid may be located at the chamber's opening **632** such as to prevent the tablet(s) **60** from entering the sample cell **621** when the sealing door **634** is open.

According to some embodiments, in order to enable unmanipulated SP water to be channeled directly from the SP into the sample cell **621** and measured there before the reagent is introduced thereto, a filling piping **651a** manifold and controllable valve(s) of faucet(s) may be used, such as manifold valve **652a,** such that when a non-manipulation sampling and measuring is to be conducted, the (electronically controllable) manifold valve **652a** is controlled to only direct SP water to pass through the pipeline section **653** of the filing piping **651a** that does not pass through the reagent chamber **631** and when the reagent is to be introduced the manifold valve **652a** is controlled to have at least some portion of SP water channeled through another filling piping section **654** that passes through the reagent chamber **631** and controllably open the sealing door **634.**

According to some embodiments, it is possible to fill the sample cell and then open the door to let water come in contact with the water.

According to some embodiments, as illustrated in **Fig .6****,** one or more drainage pipes such as drainage pipe **651b** may be used along with one or more controllable valves such as valve **652b** to controllably drain water samples from the sample cell **621.**

According to some embodiments, as illustrated in **Fig .6** , the sample cell **631** also includes two transparent surfaces or windows **625a** and **625b** located opposite and optionally in parallel to one another for enabling illuminating the water sample **30** in the sample cell 21 using one or more illuminators of an illumination unit **610** of the measuring unit **600,** and detection of optical properties of the sample **30** using one or more sensors such as optical detector(s) of a corresponding detection unit **640** of the measuring unit **640.** Other implementations may include transparent surfaces that are angular to one another, using one or more optical elements such as reflectors, for directing light emanating from an illuminator or from the sample cell **621** propagated at one propagation direction to propagate at another direction angular to the first direction of propagation.

Other optical focusing, filtering, and/or collimating hardware (device(s) and/or element(s)) may be used to optimize/improve optical detection and/or illumination.

In addition, materials whose optical characteristics change as a function of the properties of the water may be deployed as part of the optical system to enhance accuracy and/or specificity of detection and/or measurement.

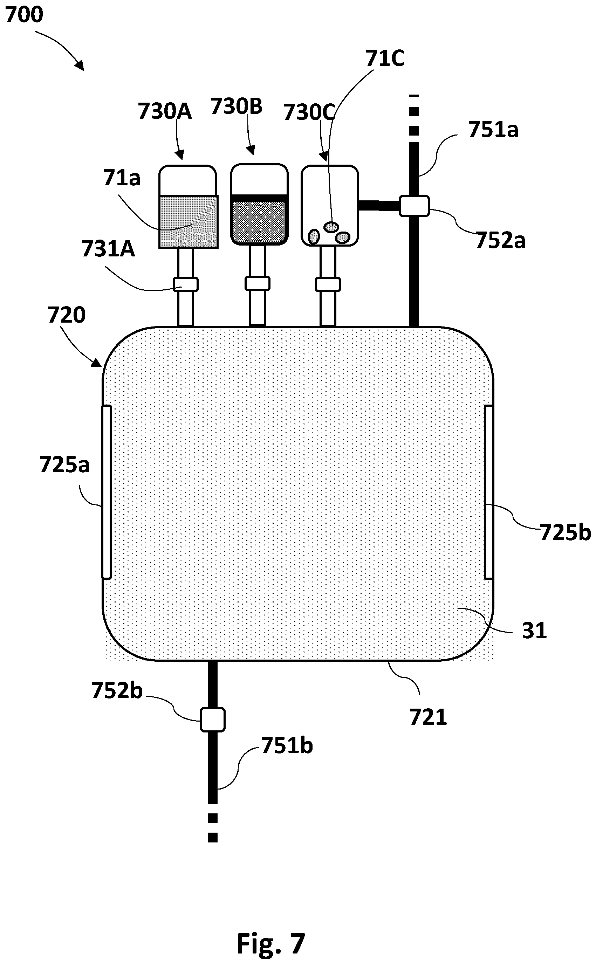

**Fig. 7** schematically illustrates another optional design for a measuring unit **700** of a LMS that uses multiple manipulation units such as manipulation units **730A-730C,** each including a dispenser for dispensing a different reagent optionally of a different reagent type, according to other embodiments.

The sample cell **731** of the sampling unit **720** of the measuring unit **700** is similar to the sample cell **620** of **Fig. 6** by also including two transparent windows **725a** and **725b** and a similar drainage mechanism including a drainage pipe **751b** and a corresponding controllable drainage valve **752b.**

Each manipulation unit **730A/730B/730C** includes a different controllable dispenser that includes a chamber for containing a respective reagent therein such as liquid reagent **71a** contained in dispenser of manipulation unit **730A** or solid dissolvable tablet(s) reagent(s) such as tablets **71c** of dispenser of the manipulation unit **730C.**

In this case, the filling pipeline **751a** can be split into a manifold such that one section of the filling pipeline **751a** enables directly channeling SP water into the sample cell **721** for obtaining the non-manipulation measurement(s) of an unmanipulated water sample **31,** and at least one filling pipeline manifold section is directed at least to dispensers of manipulation units such as unit **730C** that includes solidified tablet reagents requiring dissolving in water before dispensed into the sample cell. The filling pipeline manifold can be controlled via one or more controllable valves **752a.**

The dispensing at least of the liquid reagent(s) may be controllably conducted by using one or more dispenser-valves or faucets such as faucet **731A.**

### EXAMPLES

Example 1 is a method for monitoring at least one swimming pool (SP), the method comprising at least:
providing a detection unit that comprises at least one sensor for measuring one or more properties of water of the specific SP;
providing at least one processing unit, configured at least to receive and analyze sensor data from the detection unit for determining one or more characteristics of the SP;
obtaining a non-manipulation data of the SP water, before applying any manipulation to water of the SP, by measuring one or more properties of the SP water or at least one sample of the SP water, before it has been manipulated, using the detection unit;
applying at least one manipulation over the SP water or at least one sample from the SP water, using at least one corresponding manipulation unit, the at least one manipulation including at least introducing of at least one reagent to the SP water or to at least one sample thereof;
obtaining manipulation data of the SP water, using the detection unit, indicative of a response of the SP water to the manipulation; and
receiving and analyzing the non-manipulation data and the manipulation data of the specific manipulation, at least for determining one or more characteristics of the specific SP.

In example 2, the subject matter of example 1 may include, where the at least one manipulation is used for one or more of:
determining concentration of one or more substances in the SP water;
determining relations and/or relationship curves between one or more of SP water properties and one or more other SP water properties for the specific SP;
calibration and/or adjustment of one or more values of one or more SP parameters, associated with one or more SP properties, that are used for analyzing sensor data arriving from the detection unit, relating to the specific SP;
determining level and/or value of one or more parameters associated with water quality;
calibration and/or adjustment of one or more parameters values, thresholds and/or relations indicative of expected normal behavior of the specific SP.

In example 3, the subject matter of example 2 may include, where the determination of concentration of one or more substances includes determining concentration of at least one of the following: concentration of one or more chlorine species; concentration of cyanuric acid; concentration of calcium; concentration of biomass.

In example 4, the subject matter of any one or more of examples 2 to 3 may include, where the determination of level and/or value of one or more parameters includes at least one of: pH level; alkalinity level, Free Chlorine level, Combined Chlorine level, Oxidation Reduction Potential (ORP) level, turbidity level, water hardness level.

In example 5, the subject matter of any one or more of examples 1 to 4 may include, where the one or more reagents comprise one or more of: one or more dechlorination reagents of one or more types; an acid of one or more acid types and/or of one or more concentrations; one or more chlorination reagents of one or more types; one or more chlorine species; one or more dye substances; one or more titrants; one or more buffering substances;; one or more halochromic materials; one or more gassing and/or degassing materials.

In example 6, the subject matter of example 5, where the one or more dechlorination reagents include: Barium Sulfite and/or Calcium Sulfite.

In example 7, the subject matter of example 6 may include, where the one or more dechlorination reagents are in a form of dissoluble tablets.

In example 8, the subject matter of any one or more of examples 1 to 7 may include, where the at least one manipulation further comprises one or more of:
actively heating of at least one sample of the swimming pool water for evaporating one or more substances from the at least one sample or for water evaporation and residue measurements;
actively or passively cooling of at least one sample of the SP water;
gassing and/or degassing at least one portion or at least one sample of the swimming pool water;
applying a physical force over the SP water or at least one sample thereof;
dying of the SP water or at least one sample thereof after the at least one reagent has been introduced to the SP water or at least one sample thereof.

In example 9, the subject matter of any one or more of examples 1 to 8 may include, where each manipulation of the SP water is done by using at least one sampling unit comprising at least one sample cell for containing water samples therein, a filling mechanism for filling the sample cell with SP water and a drainage mechanism for draining water samples from the sample cell.

In example 10, the subject matter of example 9 may include, where at least one sample cell of the sampling unit is at least partially transparent to enable illumination of a water sample contained therein and to enable optical detection of the water sample.

In example 11, the subject matter of any one or more of examples 1 to 10 may include, where the at least one sensor of the detection unit comprises at least one of: at least one optical detector, at least one pH sensor, at least one pressure sensor, at least one ultrasonic (US) transducer, at least one electrochemical detector, at least one spectrometer, at least one camera, at least one pixelated optical detector, at least one halochromic sensor, at least one Oxidation Reduction Potential (ORP) sensor.

In example 12, the subject matter of any one or more of examples 1 to 11 may include, where the method further comprises one or more of the following steps:
providing an illumination unit comprising at least one light source positioned and configured to illuminate a sample of the SP water held in a sampling unit's sample cell;
channeling a sample from the SP water into the sample cell of the sampling unit;
illuminating the water sample contained by the sample cell, using at least one illuminator of the illumination unit, where the obtaining of non-manipulation and manipulation data is done by detecting optical properties of the water sample using one or more optical sensors of the detection unit.

In example 13, the subject matter of example 12 may include, where the at least one illuminator of the illumination unit, is configured to output light that is within one or more of the following spectral ranges: ultraviolet (UV), Visible (VIS).

In example 14, the subject matter of any one or more of examples 1 to 13 may include, where the method further comprises administering or dispensing the at least one reagent to the swimming pool water or to a sample thereof, for manipulating thereof, using a dispensing mechanism.

In example 15, the subject matter of any one or more of examples 1 to 13 may include, where the introducing of the at least one reagent to the SP water or at last one sample thereof comprises channeling water from the SP to a sample cell, while directing the channeled SP water to pass through a filtering unit comprising one or more reagents before the SP water enters the sample cell.

In example 16, the subject matter of any one or more of examples 1 to 15 may include, where the method further comprises determining or retrieving data associated with a suspension period for each particular manipulation being carried out, where the obtainment of the manipulation data, done by measuring the SP water or at least one sample thereof, is done after or during the suspension period, where the suspension period is associated with the at least one reagent being used and/or with a ratio between the quantity of the at least reagent being used and the quantity of SP water being tested, and/or with known concentration and/or dissolving rate of the specific at least one reagent being used for the specific manipulation.

In example 17, the subject matter of example 16 may include, where the method further includes one or more of:
mixing of a sample of the SP water with the at least one reagent, using at least one mixing mechanism;
heating or gassing/bubbling of the SP water or at least one sample thereof when the at least one reagent is introduced thereto, for causing a desired chemical reaction between the SP water and the at least one reagent being introduced, and/or for achieving a desired reaction quality;
applying acoustic signals by using at least one acoustic transducer for mixing the reagent with the water sample;
introducing the at least one reagent to a sample of the SP water contained in a sample cell and waiting for the sampled water to passively react with the introduced at least one reagent;
using a chamber of a manipulation unit including one or more solid tablets of the reagent and channeling water from the SP through the filtering unit before entering a sample cell from which water samples characteristics are measured.

In example 18, the subject matter of any one or more of examples 1 to 17 may include, where at least one of the at least one manipulation comprises multiple manipulation stages where the one or more properties of the SP water is measured for each manipulation stage.

In example 19, the subject matter of example 18 may include, where the one or more manipulation stages comprise gradual increasing of dosage, concentration, type and/or quantity of the at least one reagent being introduced to the SP water or at least one sample thereof.

In example 20, the subject matter of any one or more of examples 1 to 19 may include, where the method further includes the step of alerting situations associated with high and/or low concentration of one or more substances in the swimming pool water and/or with inappropriate levels of one or more SP parameters.

In example 21, the subject matter of any one or more of examples 1 to 20 may include, where at least one of the following steps is performed automatically: each manipulation, obtainment of the non-manipulation and manipulation data, analysis of the pre-manipulating and manipulating data, transmission of alerts, calibration and/or adjustment of analysis related parameters of the specific SP.

In example 22, the subject matter of any one or more of examples 1 to 21 may include, where the method further includes the step of managing a group of multiple swimming pools (SPs) by measuring and analyzing properties of each SP in the group of SPs, accumulating non-manipulation and manipulation data for each SP and results of analysis of this data and using the accumulated data to determine various SP maintenance and/or calibration properties for each SP in the group of SPs, by using multiple local monitoring systems, one per each SP of the group of SPs and at least one central remote monitoring system, configured to receive and analyze monitoring data from each of the local monitoring systems, where the central remote monitoring system is further configured to control monitoring and/or maintenance of each of the SP of the group of SPs via its corresponding local monitoring system.

Example 23 is a system for monitoring water quality related characteristics of water of a swimming pool (SP), the system may include at least:
a detection unit comprising one or more sensors, the detection unit being configured for measuring one or more properties of the SP water;
a manipulation unit configured to apply one or more manipulations over the SP water or part thereof, by adding at least one reagent to water from the SP, at each manipulation-session; and
at least one control and processing unit configured to receive and analyze sensor data arriving at least from the detection unit, where the detection unit and the at least one control and processing unit are configured to:
measure the SP water before applying any manipulation to the SP water, for obtaining non-manipulation data of the SP water, indicative of non-manipulation properties of the SP water, using the detection unit;
measure the SP water after and/or during its manipulation at least for obtaining manipulation data of the SP water; and
analyze the non-manipulation data and the manipulation data of the specific manipulation, at least for determining one or more characteristics of the specific SP.

In example 24, the subject matter of example 23 may include, where the at least one manipulation comprises at least dechlorination of the SP water by adding to the SP water at least one dosage of a dechlorinating reagent of at least one type, at least for determination of concentration of one or more chlorine species in the SP water.

In example 25, the subject matter of any one or more of examples 23 to 24 may include, where the analysis of the non-manipulation and manipulation data is done for at least one of the following purposes:
determining concentration of one or more other substances in the SP water;
determine level and/or value of one or more parameters associated with water quality;
calibrating and/or adjusting one or more values of one or more pool characteristics, used for analyzing sensor data arriving from the detection unit, relating to the specific SP; and/or
calibrating and/or adjusting one or more curves and/or tables of values of one or more pool-characteristics, used for analyzing sensor data arriving from the detection unit.

In example 26, the subject matter of any one or more of examples 23 to 25 may include, where the one or more sensors of the detection unit comprises at least one optical detector.

In example 27, the subject matter of example 26 may include, where at least one optical detector comprises a spectral detector configured for measuring absorption characteristics of the water from the SP.

In example 28, the subject matter of any one or more of examples 23 to 27 may include, where the system further includes a sampling unit that includes at least one sample cell, configured to obtain and hold therein a water sample sampled from the water of the SP, for conducting all measurements and manipulations over one or more water samples when held within the sample cell.

In example 29, the subject of example 28 may include, where the sample cell is at least partially transparent to enable illumination of the water sample and to enable optical detection of the obtained water sample, when held inside the sample cell.

In example 30, the subject matter of any one or more of examples 28 to 29 may include, where the at least one sampling unit is also configured for drainage of water samples therefrom.

In example 31, the subject matter of any one or more of examples 28 to 30 may include, where the system further includes one or more mixing mechanisms configured for mixing the reagent with a water sample obtained from the SP r after it has been administered or dispensed thereto.

In example 32, the subject matter of any one or more of examples 23 to 31 may include, where the system further includes an illumination unit comprising at least: at least one light source, positioned and configured to illuminate the SP water.

In example 33, the subject matter of any one or more of examples 23 to 32 may include, where the obtaining of the non-manipulation and manipulation data is done by measuring optical absorption properties of the SP water before and after and/or during it has been manipulated.

In example 34, the subject matter of example 33 may include, where the at least one light source is configured to illuminate the SP water in light that is within one of more of the following spectral ranges: ultraviolet (UV), Visible (VIS).

In example 35, the subject matter of any one or more of examples 23 to 34 may include, where the at least one reagent comprises one of: Barium Sulfite, Calcium Sulfite, Sodium Sulfite, one or more chlorine species, one or more acids, one or more dye substances, one or more buffering substances.

In example 36, the subject matter of any one or more of examples 23 to 35 may include, where the one or more water quality characteristics further comprise one or more of: concentration of one or more chlorine species, alkalinity level; pH value; cyanuric acid concentration; biomass concentration, turbidity level.

In example 37, the subject matter of any one or more of examples 23 to 36 may include, where the at least one reagent used for dechlorination manipulation is solid configured to dissolve in water.

In example 38, the subject matter of any one or more of examples 23 to 37 may include, where the system further includes at least one reagent introduction unit configured for introducing of at least one reagent to the SP water, for manipulating thereof.

In example 39, the subject matter of any one or more of examples 23 to 38 may include, where the at least one processing unit is further configured to determine and/or retrieve data associated with a suspension period for each particular manipulation being carried out, where the post-manipulation measurement for the specific manipulating being carried out, is only done after the determined suspension period, where the suspension period is associated with the reagent being used and/or with a ratio between the dosage being used and the amount of SP water being tested.

In example 40, the subject matter of any one or more of examples 23 to 39 may include, where the detection unit and/or the at least one control and processing unit are configured to measure water from the SP when manipulated at different levels of chlorination and assessing concentration of cyanuric acid in the SP water, based on the measured different levels of chlorine species of the SP water.

In example 41, the subject matter of example 40 may include, where the measuring of the SP water at different levels of chlorination is done by adding different doses of a dechlorinating reagent or alternatively chlorinating agents to one or more samples of water from the SP, and measuring absorption characteristics of the SP water at the different chlorination levels, when being illuminated, using at least one spectral detector of the detection unit and at least one illuminator.

In example 42, the subject matter of any one or more of examples 23 to 41 may include, where the system further includes a main controller, operatively associated with all units and modules of the system and with the at least one control and processing unit, for one or more of:
receiving manipulation related data from multiple SPs;
analyzing received manipulation data from the multiple SPs;
determining a maintenance plan for each SP based on per SP manipulation data; and/or
controlling monitoring and/maintenance of each of the SPs;
accumulate multiple SP monitoring data and adjust monitoring and/or maintenance of each SP, based on analysis of accumulated monitoring data.

In example 43, the subject matter of example 42 may include, where the main controller and/or the at least one control and processing unit is further configured to detect alerting situations associated with high and/or low concentration of one or more substances in the SP water and display and/or send one or more alerting notifications to one or more end devices.

In example 44, the subject matter of any one or more of examples 23 to 43 may include, where the system is configured at least for automatic and/or real time and/or near real time measuring, reagents addition, manipulation and processing.

In example 45, the subject matter of any one or more of examples 23 to 44 may include, where the system is configured for automatic sampling of a water sample into at least one sample cell for each manipulation and testing to be performed thereby, and for automatic emptying of each sample cell from each water sample, once the measuring of the water sample is completed.

In example 46, the subject matter of any one or more of examples 23 to 45 may include, where the system further includes at least one controllable reagent introduction unit locatable and configured such as to controllably expose SP water passed therethrough to dissolvable reagent.

In example 47, the subject matter of example 46 may include, where the at least one controllable reagent introduction unit comprises a sealable container comprising one or more reagents located therein, a grid barrier located at an opening of the sealable container and a controllable door for controllably sealing the opening of the container, for controllably exposing SP water passed through the sealable container to the at least one dissolvable reagent by opening the door allowing SP water to enter the container and react to the at least one reagent located therein.

While the invention has been described with respect to a limited number of embodiments, these should not be construed as limitations on the scope of the invention, but rather as exemplifications of some of the embodiments.

In the above Description, unless otherwise stated, adjectives such as "substantially" and "about" that modify a condition or relationship characteristic of a feature or features of an embodiment of the invention, are to be understood to mean that the condition or characteristic is defined to within tolerances that are acceptable for operation of the embodiment for an application for which it is intended.

It should be understood, that where the claims or specification refer to "a" or "an" element, component, object, property, characteristic and/or feature, such reference is not to be construed as its being only one of the elements. Hence, reference to "an element" or "at least one element" for instance may also encompass "one or more elements" or "at least one element" etc.

Terms used in the singular shall also include the plural, except where expressly otherwise stated or where the context otherwise requires.

In the description and claims of the present application, each of the verbs, "comprise" "include" and "have", and conjugates thereof, are used to indicate that the object or objects of the verb are not necessarily a complete listing of components, elements or parts of the subject or subjects of the verb.

Unless otherwise stated, the use of the expression "and/or" between the last two members of a list of options for selection indicates that a selection of one or more of the listed options is appropriate and may be made. Further, the use of the expression "and/or" may be used interchangeably with the expressions "at least one of the following", "any one of the following" or "one or more of the following", followed by a listing of the various options.

It is appreciated that certain features of the invention, which are, for clarity, described in the context of separate embodiments or example, may also be provided in combination in a single embodiment. Conversely, various features of the invention, which are, for brevity, described in the context of a single embodiment, example and/or option, may also be provided separately or in any suitable sub-combination or as suitable in any other described embodiment, example or option of the invention. Certain features described in the context of various embodiments, examples and/or optional implementation are not to be considered essential features of those embodiments, unless the embodiment, example and/or optional implementation is inoperative without those elements.

It is noted that the terms "in some embodiments", "according to some embodiments", "according to some embodiments of the invention", "for example", "e.g.,", "for instance" and "optionally" may herein be used interchangeably.

The number of elements shown in the Figures should by no means be construed as limiting and is for illustrative purposes only.

Throughout this application, various embodiments may be presented in and/or relate to a range format. It should be understood, that the description in range format is merely for convenience and brevity and should not be construed as an inflexible limitation on the scope of the embodiments.

## Claims

1. A method for monitoring at least one swimming pool (SP) (1,50a-50c), the method comprising at least:
providing a detection unit (140, 640) that comprises at least one sensor for measuring one or more properties of water of the specific SP (1, 50a/50b/50c);
providing at least one processing unit (200, 350), configured at least to receive and analyze sensor data from the detection unit (140, 640) for determining one or more characteristics of the SP;
obtaining non-manipulation data of the SP water by measuring one or more properties of the SP water or at least one sample of the SP water, without it being manipulated (Step 44/53), using the detection unit (140, 640);
applying at least one manipulation over the SP water or at least one sample from the SP water, using at least one corresponding manipulation unit (130, 630, 730A-730C), the at least one manipulation including at least dechlorination of the SP water or at least one sample from the SP water;
obtaining manipulation data (Step 46, Step 55) of the SP water, using the detection unit (140, 640), indicative of a response of the SP water to the manipulation; and
receiving and analyzing the non-manipulation data and the manipulation data of the specific manipulation, at least for determining one or more chlorine species concentration in the SP water (Step 47, Step 56).

2. The method of claim 1, wherein the at least one manipulation is used for one or more of:
determining concentration of one or more substances in the SP water;
determining relations between one or more of SP water properties and one or more other SP water properties for the specific SP;
calibration and/or adjustment of one or more values of one or more SP parameters, associated with one or more SP properties, that are used for analyzing sensor data arriving from the detection unit, relating to the specific SP;
determining level and/or value of one or more parameters associated with water quality;
calibration and/or adjustment of one or more parameters values, thresholds and/or relations indicative of expected normal behavior of the specific SP.

3. The method of claim 2, wherein the determination of concentration of one or more substances further includes determining concentration of one or more of:
cyanuric acid;
calcium;
biomass of one or more types;
one or more chloramines;
one or more chloramines including one or more of: NH₂Cl, NHCl₂, NCl₃;
one or more cyanurate species;
one or more cyanurate species including one or more of: H₃Cy, H₂Cy⁻, HCy²⁻, Cy³⁻;
one or more chlorinated cyanurates;
one or more chlorinated cyanurates including one of more of:Cl3Cy, Cl2Cy⁻, HClCy⁻.

4. The method of claim 2, wherein the determination of level and/or value of one or more parameters includes at least one of: pH level; alkalinity level, Free Chlorine Level, Combined Chlorine Level, Oxidation Reduction Potential (ORP) level, turbidity level, water hardness level.

5. The method of claim 1, wherein the at least one manipulation includes introduction of one or more chemical reagent to the SP water or to one or more samples of the SP water.

6. The method of claim 5, wherein the one or more chemical reagents include one or more of:
one or more dechlorination reagents;
an acid of one or more acid types and/or of one or more concentrations;
one or more chlorination reagents of one or more types;
one or more chlorine species;
one or more dye substances;
one or more titrants;
one or more buffering substances;
one or more dye substances;
one or more halochromic materials;
one or more gassing and/or degassing materials.

7. The method of claim 1, wherein the dechlorination manipulation comprises one or more of:
introducing of one or more dechlorination reagents of one or more types into the SP water or one or more samples of the SP water;
heating of the SP water or one or more samples of the SP water;
applying pressure to the SP water or one or more samples of the SP water; and/or bubbling of the SP water or one or more samples of the SP water.

8. The method of claim 7, wherein the one or more dechlorination reagents include:
Barium Sulfite and/or Calcium Sulfite, Sodium Sulfite.

9. The method of claim 1, wherein the at least one manipulation comprises one or more of:
actively heating of at least one sample of the swimming pool water for evaporating one or more substances from the at least one sample or for water evaporation and residue measurements;
actively or passively cooling of at least one sample of the SP water;
gassing and/or degassing at least one portion or at least one sample of the swimming pool water;
applying a physical force over the SP water or at least one sample thereof;
dying of the SP water or at least one sample thereof after the at least one reagent has been introduced to the SP water or at least one sample thereof.

10. The method of claim 1, wherein the obtainment of the manipulation data is done after a reaction time associated with the specific manipulation applied to the SP water or at least one sample thereof.

11. A system (10, 310a, 310b, 310c) for monitoring water quality related characteristics of water of a swimming pool (SP), the system comprising at least:
a detection unit (140, 640) comprising one or more sensors, the detection unit being configured for measuring at least one or more properties of the SP water;
a manipulation unit (130, 630, 730A-730C) configured to apply one or more manipulations over the SP water or one or more portions thereof; and
at least one control and processing unit (200, 350) configured to receive and analyze sensor data arriving at least from the detection unit (140, 640);
wherein the detection unit (140, 640) and the at least one control and processing unit (200, 350) are configured to:
obtain non-manipulation data of the SP water by measuring one or more properties of the SP water or at least one sample of the SP water, without it being manipulated, using the detection unit;
obtain manipulation data of the SP indicative to response of the SP water to the applied manipulation, using the detection unit; and
analyze the obtained non-manipulation data and the manipulation data of the specific manipulation, at least for determining concentration of one or more chlorine species in the SP water.

12. The system (10, 310a, 310b, 310c) of claim 11, wherein the dechlorination of the SP water is done by at least one of:
introducing of a dechlorinating reagent of at least one type to the SP water or at least one sample from the SP water;
heating of the SP water or at least one sample from the SP water;
bubbling of the SP water or at least one sample from the SP water.

13. The system (10, 310a, 310b, 310c) of claim 11, wherein the analysis of the non-manipulation and manipulation data is further done for at least one of the following purposes:
determining concentration of one or more other substances in the SP water;
determine level and/or value of one or more parameters associated with water quality;
calibrating and/or adjusting one or more values of one or more pool characteristics, used for analyzing sensor data arriving from the detection unit, relating to the specific SP; and/or
calibrating and/or adjusting one or more curves and/or tables of values of one or more pool-characteristics, used for analyzing sensor data arriving from the detection unit.

14. The system (10, 310a, 310b, 310c) of claim 11 further comprising a sampling unit (120, 620, 720) that comprises at least one sample cell (121, 621, 721), each sample cell being configured to obtain and hold therein a water sample sampled from the water of the SP, for conducting all measurements and manipulations over one or more water samples when held within the at least one sample cell.

15. The system (10, 310a, 310b, 310c) of claim 11 further comprising a main controller, operatively associated with all units and modules of the system and with the at least one control and processing unit, for one or more of:
receiving and analyzing manipulation and non-manipulation data from multiple SPs;
determining a maintenance plan for each SP based on per SP manipulation data, based on analysis results; and/or
controlling monitoring and/maintenance of each of the SPs;
accumulate multiple SP monitoring data and adjust monitoring and/or maintenance of each SP, based on analysis of accumulated monitoring data.
